# EUROPEAN PATENT APPLICATION

(11) **EP 4 781 974 A2**
(43) Date of publication of application: **29.07.2026**
(21) Application number: 26185221.4
(22) Date of filing: 02.04.2020
(51) Int. Cl.: A61K 8/58

(54) **COSMETIC COMPOSITION COMPRISING A BRANCHED ALKANE AND A COMBINATION OF PARTICULAR SILICONES**

(30) Priority: 02.04.2019 FR 1903489
(62) Divisional of application: 20715069.9
(71) Applicant: L'OREAL, 75008 Paris (FR)
(72) Inventor: FRANGI, Stéphanie, 93400 SAINT-OUEN (FR); VALENTIN, Julie, 93400 SAINT-OUEN (FR)
(74) Representative: Casalonga

(57) **Abstract**

A subject of the present invention is a cosmetic composition, which is preferably anhydrous, comprising:
a) one or more branched alkanes having from 8 to 16 carbon atoms;
b) one or more dimethiconols;
c) one or more silicones, other than dimethiconols, having a viscosity at 25°C of less than 200×10⁻⁶m²/s; and
d) one or more silicones, other than dimethiconols, having a viscosity at 25°C within the range extending from 0.01 m²/s to 1 m²/s,
with preferably a weight ratio of the total amount of silicone(s) c) relative to the total amount of silicone(s) d) ranging from 0.5 to 5.

A subject of the present invention is also a cosmetic treatment process using this composition, and also the use of said composition for conditioning keratin fibres.

## Description

The present invention relates to a composition intended for the cosmetic treatment of keratin fibres, in particular of human keratin fibres such as the hair, comprising one or more branched alkanes, one or more dimethiconol(s), and also a combination of low-viscosity and high-viscosity silicones.

The invention also relates to a process for the cosmetic treatment of keratin fibres, in which such a composition is applied to said fibres, and also to the use of this composition for caring for keratin fibres.

The hair is generally damaged and weakened by the action of external atmospheric agents such as light and bad weather, and also by mechanical or chemical treatments, such as brushing, combing, dyeing, bleaching, permanent-waving and/or relaxing, or even repeated washing.

Hair is thus damaged by these various factors and may over time become dry, coarse, brittle or dull, especially in fragile areas, and more particularly at the ends.

Thus, to overcome these drawbacks, it is common practice to resort to haircare products using compositions intended to condition the hair, giving it satisfactory cosmetic properties, especially in terms of smoothness, sheen, softness, suppleness, lightness, a natural feel and good disentangling properties.

These haircare compositions, intended to be applied regularly to the hair, may be, for example, hair conditioners, masks or sera, and may be in the form of gels, hair lotions or care creams that are more or less thick.

It is in particular known practice to use compositions in the form of oily sera, containing combinations of silicone oils or non-silicone oils.

However, such compositions still too often result in excessive greasing of keratin fibres, which results in said fibres having a greasy or even tacky feel, and a lank, charged visual appearance.

Furthermore, compositions containing silicone oils, once applied to the hair, frequently result in effects that are not very pleasant to the touch, in particular the hair has a tendency to be coarse and/or to squeak (that is to say produce an unpleasant sound), in particular when the strands of hair are rubbed together while sliding the fingers along the hair from the root to the end.

Finally, the viscosity of these compositions is not always optimal, and does not make it possible to package them in a pump dispenser bottle.

The present invention aims to provide compositions which do not have the drawbacks mentioned above, and which are capable of conditioning keratin fibres in a long-lasting manner, in particular by giving them good cosmetic properties.

This aim is achieved by the present invention, a subject of which is in particular a cosmetic composition, which is preferably anhydrous, comprising:
a) one or more branched alkanes having from 8 to 16 carbon atoms;
b) one or more dimethiconols;
c) one or more silicones, other than dimethiconols, having a viscosity at 25°C of less than 200×10⁻⁶m²/s (200 cSt); and
d) one or more silicones, other than dimethiconols, having a viscosity at 25°C within the range extending from 0.01 m²/s to 1 m²/s;
with preferably a weight ratio of the total amount of silicone(s) c) relative to the total amount of silicone(s) d) ranging from 0.5 to 5.

The composition according to the invention makes it possible to condition keratin fibres very satisfactorily. In particular, it makes it possible to significantly improve the sheen, the softness, the ease of disentangling of the hair, without making it lank or making it greasy. The keratin fibres remain light, supple, and easy to style and to shape. They are neither visually greasy nor greasy to the touch, and do not squeak when touched.

This composition also makes it possible to nourish and repair damaged hair, in particular at the ends, and to facilitate the styling and straightening of curly or kinky hair.

Finally, the composition according to the invention has a suitable viscosity, for making it possible to package it in a pump dispenser bottle. It is neither too fluid nor too viscous, and can be dispensed by a pump system.

A subject of the invention is also a process for the cosmetic treatment of keratin fibres, in which the composition according to the invention is applied to the keratin fibres.

Another subject of the present invention relates to the use of the composition as defined previously for the conditioning of keratin fibres, in particular human keratin fibres such as the hair.

Other subjects, characteristics, aspects and advantages of the invention will emerge even more clearly on reading the description and the example that follows.

In the present description, and unless otherwise indicated:
- the expression "at least one" is equivalent to the expression "one or more" and can be replaced therewith;
- the expression "between" is equivalent to the expression "ranging from" and can be replaced therewith, and implies that the limits are included.
- According to the present application, the term "keratin fibres" denotes human keratin fibres and more particularly the hair.

Throughout the text hereinbelow, the term "silicone" denotes, in accordance with what is generally accepted, any organosilicon polymer or oligomer of linear or cyclic, branched or crosslinked structure, of variable molecular weight, obtained by polymerization and/or by polycondensation of suitably functionalized silanes, and formed essentially from a repetition of main units in which the silicon atoms are linked together via oxygen atoms (siloxane bond -Si-O-Si-), optionally substituted hydrocarbon-based groups being directly linked via a carbon atom to said silicon atoms. The hydrocarbon-based groups that are the most common are alkyl groups, notably C₁-C₁₀ alkyl groups and in particular methyl, fluoroalkyl groups, the alkyl part of which is C₁-C₁₀, and aryl groups such as in particular phenyl groups.

In a manner known per se, the viscosity of the silicones, which is the kinematic viscosity, is measured at 25°C and at atmospheric pressure (1 atm, 1.013×10⁵ Pa) according to standard ASTM 445 Appendix C.

The weight-average molecular weights of the silicones may be measured by gel permeation chromatography (GPC) at ambient temperature (25°C), as polystyrene equivalent. The columns used are µ styragel columns. The eluent is THF and the flow rate is 1 ml/min. 200 µl of a 0.5% by weight solution of silicone in THF are injected. Detection is performed by refractometry and UV-metry.

### The branched alkane(s) a):

The composition according to the present invention contains one or more branched alkanes comprising from 8 to 16 carbon atoms, preferably from 10 to 16 carbon atoms, more preferentially from 10 to 14 carbon atoms.

For the purposes of the invention, the term "alkane" is intended to mean a saturated linear compound formed solely from carbon and hydrogen atoms.

The branched alkanes according to the invention correspond to the formula CₙH₂ₙ₊₂ with n being an integer ranging from 8 to 16, preferably from 10 to 16, more preferentially from 10 to 14.

These compounds can advantageously be chosen from isohexadecane, isododecane, isodecane and isooctane.

Particularly preferably, the branched alkane(s) according to the invention comprise(s) 12 carbon atoms. A particularly preferred compound is isododecane.

The branched alkane(s) a) are advantageously present in a total content of greater than or equal to 20% by weight relative to the total weight of the composition.

According to one preferred embodiment, the branched alkane(s) a) are present in a total content ranging from 30 to 95% by weight, better still from 35 to 90% by weight, even better still from 40 to 85% by weight, relative to the total weight of the composition.

According to one particularly preferred embodiment, the composition according to the invention contains isododecane, in a content ranging from 30 to 95% by weight, better still from 35 to 90% by weight, even better still from 40 to 85% by weight, relative to the total weight of the composition.

### The dimethiconol(s) b):

The name dimethiconol denotes, in a manner known per se, polydimethylsiloxanes comprising dimethylsilanol end groups.

Preferably, the dimethiconol(s) according to the invention has (have) a weight-average molecular weight (Mw) of greater than 1000 daltons, preferably greater than 10 000 daltons, more preferentially greater than 50 000 daltons, better still greater than 100 000 daltons.

By way of known compounds, mention may in particular be made of the compounds having the INCI name dimethiconol proposed by the company Dow Corning under the trade names Dow Corning 1515 Gum and Dowsil 1515 Gum.

The dimethiconol(s) are advantageously present in a total content ranging from 0.5 to 15% by weight, better still from 1 to 10% by weight, even better still from 1.5 to 8% by weight, relative to the total weight of the composition.

### The silicone(s) c):

The composition according to the invention contains one or more silicones having a viscosity at 25°C of less than 200×10⁻⁶m²/s (200 cSt).

Preferably, the viscosity of the silicone(s) c) is less than or equal to 180×10⁻⁶m²/s, more preferentially less than or equal to 150×10⁻⁶m²/s, and even more preferentially less than or equal to 120× 10⁻⁶m²/s.

The silicone(s) c) are other than dimethiconols, that is to say that they are not polydimethylsiloxanes comprising dimethylsilanol end groups.

The silicone(s) c) are more particularly chosen from volatile silicones.

For the purposes of the present invention, the term "volatile silicone" is intended to mean a silicone that is capable of evaporating on contact with the skin or the hair in less than one hour, at ambient temperature and at atmospheric pressure. Such a silicone has in particular a saturation vapour pressure ranging from 0.13 to 40 000 Pa (10⁻³ to 300 mmHg), in particular ranging from 1.3 to 13 000 Pa (0.01 to 100 mmHg), and more particularly ranging from 1.3 to 1300 Pa (0.01 to 10 mmHg).

The silicone(s) c) are more preferentially chosen from those with a boiling point ranging from 60°C to 260°C, and even more particularly from:
c1) linear silicones having from 2 to 9 silicon atoms.

Said silicones preferably have a viscosity of less than or equal to 6×10⁻⁶m²/s at 25°C (6 cSt), more preferentially less than or equal to 5×10⁻⁶m²/s at 25°C (5 cSt).

Mention may for example be made of heptamethylhexyltrisiloxane, heptamethyloctyltrisiloxane, hexamethyldisiloxane, octamethyltrisiloxane, decamethyltetrasiloxane, dodecamethylpentasiloxane.

c2) linear polydimethylsiloxanes having in particular more than 9 silicon atoms.

Such polydimethylsiloxanes are preferably chosen from the compounds of the following formula: (CH₃)₃SiO[Si(CH₃)₂O]ₙSi(CH₃)₃ with n ranging from 8 to 100 and preferably from 8 to 20.

According to one preferred embodiment, the silicone(s) c) are chosen from linear polydimethylsiloxanes c2) having more than 9 silicon atoms.

Preferably, the silicone(s) c) has (have) a weight-average molecular weight (Mw) of less than 2000 daltons, preferably less than 1000 daltons.

The silicone(s) c) are advantageously present in a total content ranging from 1 to 50% by weight, better still from 2 to 45% by weight, and even better still from 5 to 40% by weight, relative to the total weight of the composition.

Preferably, the composition according to the invention is free of cyclomethicone, that is to say of cyclic polydimethylsiloxane, in particular of cyclomethicone comprising from 4 to 6 silicon atoms.

The expression "free of cyclomethicone" is intended to mean that the composition according to the invention does not comprise cyclomethicone, or that, when the composition according to the invention contains one or more cyclomethicone(s), the latter are present in a total content of less than or equal to 0.1% by weight, preferably less than 0.05% by weight, relative to the total weight of the composition, even better still the composition is totally free of cyclomethicone (0% by weight).

Preferably, the composition according to the invention is free of cyclopentasiloxane(s).

The expression "free of cyclopentasiloxane(s)" is intended to mean that the composition according to the invention does not comprise cyclopentasiloxane(s), or that, when the composition according to the invention contains one or more cyclopentasiloxane(s), the latter are present in a total content of less than or equal to 0.1% by weight, preferably less than 0.05% by weight, relative to the total weight of the composition, even better still the composition is totally free of cyclopentasiloxane (0% by weight).

According to one particularly preferred embodiment, the composition according to the invention is free of decamethylcyclopentasiloxane.

The expression "free of decamethylcyclopentasiloxane" is intended to mean that the composition according to the invention does not comprise decamethylcyclopentasiloxane, or that, when the composition according to the invention contains decamethylcyclopentasiloxane, this compound is present in a total content of less than or equal to 0.1% by weight, preferably less than 0.05% by weight, relative to the total weight of the composition, even better still the composition is totally free of decamethylcyclopentasiloxane (0% by weight).

### The silicone(s) d):

The composition according to the invention contains one or more silicones having a viscosity at 25°C within the range extending from 0.01 m²/s to 1 m²/s.

Preferably, the viscosity of the silicone(s) d) is within the range extending from 0.01 m²/s (10 000 cSt) to 0.8 m²/s (800 000 cSt), better still from 0.01 m²/s (10 000 cSt) to 0.7 m²/s (700 000 cSt), even better still from 0.2 m²/s (200 000 cSt) to 0.6 m²/s (600 000 cSt).

Preferably, the silicone(s) d) are liquid at 25°C and at atmospheric pressure (1.013×10⁵ Pa).

The silicone(s) d) are other than dimethiconols, that is to say that they are not polydimethylsiloxanes comprising dimethylsilanol end groups.

The silicone(s) d) are more particularly chosen from non-volatile silicones. For the purposes of the present invention, the term "non-volatile silicone" is intended to mean a silicone with a saturation vapour pressure of less than 0.13 Pa (0.01 mmHg).

Preferably, the silicone(s) d) have a weight-average molecular weight (Mw) of greater than 50 000 daltons, more preferentially ranging from 100 000 to 500 000 daltons, and better still ranging from 200 000 to 400 000 daltons.

The silicone(s) d) are more preferentially chosen from polydialkylsiloxanes, and in particular from polydimethylsiloxanes comprising trimethylsilyl end groups, also known under the names dimethicone and PDMS.

Mention may be made, among these polydialkylsiloxanes, in a non-limiting way, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 Fluid having a viscosity of 60 000 mm²/s, and Xiameter PMX 200 silicone fluid 500 000 cSt or 300 000 cSt;
- the Viscasil^{®} oils from General Electric and certain oils of the SF series (SF 96, SF 18) from General Electric.

According to one particularly preferred embodiment, the silicone(s) d) are chosen from polydimethylsiloxanes comprising trimethylsilyl end groups (PDMS).

The silicone(s) d) are advantageously present in a total content ranging from 1 to 30% by weight, better still from 2 to 25% by weight, even better still from 3 to 20% by weight, relative to the total weight of the composition.

According to one preferred embodiment, the composition according to the invention contains the silicone(s) c) and d) described above in amounts such that the weight ratio of the total amount of the silicone(s) c) relative to the total amount of the silicone(s) d) ranges from 0.5 to 5. Preferably, this weight ratio ranges from 0.5 to 5, more preferentially from 0.6 to 4, better still from 0.7 to 3, or even from 0.8 to 2.5 and even better still from 1 to 2.

### The composition:

According to one preferred embodiment, the composition according to the invention is anhydrous, that is to say that it comprises a water content of less than or equal to 4% by weight, preferably less than or equal to 2% by weight, more preferentially less than or equal to 1% by weight, even more preferentially less than or equal to 0.5% by weight, relative to the total weight of the composition. Preferably, the composition according to the invention is totally free of water (0%).

The composition according to the invention is advantageously clear, which gives it a particularly attractive aesthetic appearance that is highly sought after by users.

The composition according to the invention is advantageously in the form of a clear to translucent, more preferentially clear, fluid.

The clarity of the composition according to the invention can be characterized by the measurement of its turbidity, by turbidimetry (in NTU units). In the context of the present invention, the turbidity measurements were carried out with a turbidimeter, model HI 88713-ISO from the company Hanna Instruments.

Preferably, the turbidity of the compositions according to the invention, measured at ambient temperature (25°C) and atmospheric pressure (1 atm), is less than 400 NTU units, better still less than 300 NTU units, in particular between 0 and 250 NTU units, or even between 0 and 200 NTU units, better still between 0 and 100 NTU units, and even better still between 1 and 80 NTU units.

Preferably, the composition according to the invention has a dynamic viscosity at ambient pressure and temperature (25°C; 1 atm) of less than 1000 mPa/s, in particular ranging from 80 to 500 mPa/s, or even from 90 to 400 mPa/s, better still from 100 to 300 mPa/s.

The dynamic viscosity can be measured using an MCR 502 rotational rheometer from Anton Paar, with a cone/plate diameter 50 mm / 1° (sanded steel at 5 µm) geometry; rotational speed 200 revolutions/min; measurements carried out at 25°C, 1 atm.

The composition according to the invention may also contain additives such as, for example, fragrances, and fatty substances other than the compounds a), b), c) and d) described above.

These additives may be present in the composition according to the invention in an amount ranging from 0% to 5% by weight relative to the total weight of the composition.

Those skilled in the art will take care to select these optional additives and the amounts thereof so that they do not harm the properties of the compositions of the present invention.

The composition according to the invention is advantageously packaged in a non-aerosol device, such as in particular a pump dispenser bottle.

### The cosmetic treatment process:

A subject of the present invention is also a process for the cosmetic treatment of human keratin fibres such as the hair, comprising the application to said fibres of a composition as described above.

The composition may be applied to dry or wet keratin fibres that have optionally been washed with a shampoo. Preferably, the composition according to the invention is applied to wet keratin fibres.

According to one embodiment, the keratin fibres are then rinsed with water, and optionally washed with a shampoo and then rinsed with water, before being dried or left to dry.

In this embodiment, the composition according to the invention is applied for a leave-on time that may range from 1 to 15 minutes and preferably from 2 to 10 minutes.

According to one preferred embodiment, the keratin fibres are not rinsed after application of the composition. They can then undergo heat and/or mechanical treatment for drying and/or shaping, for example straightening by means of a heated iron.

### The use:

The present invention also relates to the use of a composition as described above, for conditioning keratin fibres, in particular human keratin fibres such as the hair.

The composition can be used on wet or dry hair, in rinse-off or leave-on mode, and preferably in leave-on mode (that is to say that the keratin fibres are not rinsed after application of the composition).

The examples that follow serve to illustrate the invention without, however, being limiting in nature.

### EXAMPLES

In the examples that follow, all the amounts are given, unless otherwise indicated, as mass percentages of active material relative to the total weight of the composition.

The viscosity of the compositions was measured using an MCR 502 rotational rheometer from Anton Paar, with a cone/plate diameter 50 mm / 1° (sanded steel at 5 µm) geometry; rotational speed 200 revolutions/min; measurements carried out at 25°C, 1 atm.

### Example 1:

The following compositions were prepared from the ingredients of which the contents are indicated in the table below.

**[Table 1]**

| Composition | A | B | C | D |
|---|---|---|---|---|
| Dimethicone with a viscosity of 500 000 cSt (1) | 10 | 5 | 10 | 10 |
| Dimethicone with a viscosity of 5 cSt (2) | 10 | 5 | 10 | 10 |
| Dimethicone with a viscosity of 2 cSt (3) | | | 10 | |
| Dimethiconol (4) | 4 | 7.5 | 5 | 5 |
| Isododecane | 76 | 82.5 | 65 | 75 |
| Silicone c / silicone d weight ratio | 1 | 1 | 2 | 1 |
| Dynamic viscosity of the composition | 125 | 174 | 178 | 173 |

| | | | | |
|---|---|---|---|---|
| (1) Sold under the name Xiameter PMX-200 Silicone fluid 500 000 cSt by Dow Corning (2) Sold under the name Xiameter PMX-200 Silicone fluid 5 cSt by Dow Corning (3) Sold under the name Xiameter PMX-200 Silicone fluid 2 cSt by Dow Corning (4) Sold under the name Dow Corning 1515 Gum by Dow Corning | | | | |

All the compositions A to D are perfectly clear (turbidity = 0 or 1). These compositions, after application to the hair, leave a clean and non-tacky feel and supple hair.

### Example 2:

The following composition was prepared from the ingredients of which the contents are indicated in the table below.

**[Table 2]**

| Composition | E |
|---|---|
| Dimethicone with a viscosity of 300 000 cSt (1) | 17.5 |
| Dimethicone with a viscosity of 100 cSt (2) | 22.5 |
| Dimethicone with a viscosity of 5 cSt (3) | 12 |
| Dimethiconol (4) | 1.5 |
| Isododecane | qs 100% |
| Silicone c / silicone d weight ratio | 2 |
| Dynamic viscosity of the composition | 226 |

| | |
|---|---|
| (1) Sold under the name Xiameter PMX-200 Silicone fluid 300 000 cSt by Dow Corning (2) Sold under the name Xiameter PMX-200 Silicone fluid 100 cSt by Dow Corning (3) Sold under the name Xiameter PMX-200 Silicone fluid 5 cSt by Dow Corning (4) Sold under the name Dow Corning 1515 Gum by Dow Corning | |

Composition E is perfectly clear (turbidity = 0 or 1). This composition, after application to the hair, leaves a clean and non-tacky feel and supple hair.

### Comparative Example 3:

The following comparative composition K was prepared from the ingredients of which the contents are indicated in the table below.

**[Table 3]**

| Composition | K |
|---|---|
| Dimethicone with a viscosity of 500 000 cSt (1) | - |
| Dimethicone with a viscosity of 5 cSt (2) | - |
| Dimethicone with a viscosity of 100 cSt (3) | 25 |
| Dimethiconol (4) | 10 |
| Isododecane | 65 |
| Silicone c / silicone d weight ratio | undefined |
| Dynamic viscosity of the composition | 349 |

| | |
|---|---|
| (1) Sold under the name Xiameter PMX-200 Silicone fluid 500 000 cSt by Dow Corning (2) Sold under the name Xiameter PMX-200 Silicone fluid 5 cSt by Dow Corning (3) Sold under the name Xiameter PMX-200 Silicone fluid 100 cSt by Dow Corning (4) Sold under the name Dow Corning 1515 Gum by Dow Corning | |

This composition is perfectly clear (turbidity = 0 or 1). However, after application to the hair, it leaves an unclean visual appearance, the head of hair is tacky in the hand, and stiff.

## Claims

1. Cosmetic composition comprising:
a) one or more branched alkanes having from 8 to 16 carbon atoms;
b) one or more dimethiconols;
c) one or more silicones, other than dimethiconols, having a viscosity at 25°C of less than 200×10⁻⁶m²/s; and
d) one or more silicones, other than dimethiconols, having a viscosity at 25°C within the range extending from 0.01 m²/s to 1 m²/s.

2. Composition according to the preceding claim, **characterized in that** said branched alkane(s) is (are) chosen from branched alkanes having from 10 to 16 carbon atoms, preferably from 10 to 14 carbon atoms, and more preferentially 12 carbon atoms.

3. Composition according to either one of the preceding claims, **characterized in that** the branched alkane(s) a) are present in a total content of greater than or equal to 20% by weight, preferably ranging from 30 to 95% by weight, more preferentially from 35 to 90% by weight, and even better still from 40 to 85% by weight, relative to the total weight of the composition.

4. Composition according to any one of the preceding claims, **characterized in that** the dimethiconol(s) are present in a total content ranging from 0.5 to 15% by weight, better still from 1 to 10% by weight, even better still from 1.5 to 8% by weight, relative to the total weight of the composition.

5. Composition according to any one of the preceding claims, **characterized in that** the viscosity of the silicone(s) c) is less than or equal to 180×10⁻⁶m²/s, more preferentially less than or equal to 150×10⁻⁶m²/s, and even more preferentially less than or equal to 120×10⁻⁶m²/s.

6. Composition according to any one of the preceding claims, **characterized in that** the silicone(s) c) are chosen from:
c1) linear silicones having from 2 to 9 silicon atoms; and
c2) linear polydimethylsiloxanes having more than 9 silicon atoms;
and preferably from linear polydimethylsiloxanes having more than 9 silicon atoms.

7. Composition according to any one of the preceding claims, **characterized in that** the silicone(s) c) are present in a total content ranging from 1 to 50% by weight, preferably from 2 to 45% by weight and more preferentially from 5 to 40% by weight relative to the total weight of the composition.

8. Composition according to any one of the preceding claims, **characterized in that** it is free of decamethylcyclopentasiloxane, and preferably free of cyclopentasiloxane(s), and even more preferably is free of cyclomethicone.

9. Composition according to any one of the preceding claims, **characterized in that** the viscosity of the silicone(s) d) is within the range extending from 0.01 m²/s to 0.8 m²/s, preferably from 0.01 m²/s to 0.7 m²/s, and even better still from 0.2 m²/s to 0.6 m²/s.

10. Composition according to any one of the preceding claims, **characterized in that** the silicone(s) d) are chosen from polydialkylsiloxanes, preferably from polydimethylsiloxanes comprising trimethylsilyl end groups.

11. Composition according to any one of the preceding claims, **characterized in that** the silicone(s) d) are present in a total content ranging from 1 to 30% by weight, better still from 2 to 25% by weight and even better still from 3 to 20% by weight, relative to the total weight of the composition.

12. Composition according to any one of the preceding claims, **characterized in that** the weight ratio of the total amount of the silicone(s) c) relative to the total amount of the silicone(s) d) ranges from 0.5 to 5, more preferentially from 0.6 to 4, better still from 0.7 to 3, or even from 0.8 to 2.5 and even better still from 1 to 2.

13. Composition according to any one of the preceding claims, **characterized in that** it is anhydrous.

14. Process for the cosmetic treatment of human keratin fibres such as the hair, comprising the application to said keratin fibres of a composition as defined in any one of the preceding claims.

15. Use of a composition as defined in any one of Claims 1 to 13, for conditioning keratin fibres, in particular human keratin fibres such as the hair.
